Europäisches Patentamt

European Patent Office

Office européen des brevets

(19)

(11) Publication number: **0 194 547**

**A2**

# (12) EUROPEAN PATENT APPLICATION

(21) Application number: **86102771.2**

(22) Date of filing: **03.03.86**

(51) Int. Cl.⁴: **A 61 K 31/615**
**A 61 K 9/20**

(30) Priority: **15.03.85 US 712191**

(43) Date of publication of application:
**17.09.86 Bulletin 86/38**

(84) Designated Contracting States:
**AT BE CH DE FR GB IT LI NL**

(71) Applicant: **MILES LABORATORIES, INC.**
**1127 Myrtle Street**
**Elkhart Indiana 46514(US)**

(72) Inventor: **Duvall, Ronald N.**
**2344 Brookwood Drive**
**Elkhart Indiana 46514(US)**

(72) Inventor: **Lipka, Edward A.**
**22817 Banbury Cross**
**Elkhart Indiana 46514(US)**

(74) Representative: **Jesse, Ralf-Rüdiger, Dr. et al,**
**Bayer AG Konzernverwaltung RP Patentabteilung**
**D-5090 Leverkusen 1 Bayerwerk(DE)**

(54) **Stable acetylsalicylic acid and diphenhydramine citrate effervescent composition.**

(57) A substantially stable composition containing an effervescent couple, acetylsalicylic acid and diphenhydramine citrate is disclosed. Other known active agents, as well as optional ingredients such as flavorings, may also be included in the composition. The composition effervesces when placed in water The effervescent couple preferably is sodium bicarbonate and citric acid.

EP 0 194 547 A2

Croydon Printing Company Ltd

0194547

## STABLE ACETYLSALICYLIC ACID AND DIPHENHYDRAMINE
## CITRATE EFFERVESCENT COMPOSITION

The present invention relates to a novel composition of effervescent couple, acetylsalicylic acid (an analgesic) and the citrate salt of diphenhydramine, which is added to the acetylsalicylic acid as an antihistamine and/or an antitussive and its use in the production of medicaments for treating cold.

### BACKGROUND AND PRIOR ART

Medicaments employing acetylsalicylic acid (ASA) are well known. However, many other active ingredients, when used together with this analgesic in a unitary formulation, may cause instability in the acetylsalicylic acid, as a result of which undesirable reaction products such as salicylic acid may form. Salicylic acid is toxic and thus many governmental regulatory agencies have established quite low limits on the amount that may be present in a medicament. A major use of salicylic acid is as a topical keratolytic, i.e. as an agent to remove an overgrowth of horny tissue from skin. Thus, to obviate instability in a unitary formulation, the acetylsalicylic acid component is physically separated from the other active ingredients.

MS-1381

In these compositions, some means is used to keep the active ingredients in the composition from contacting one another, such as press-coating a tablet, film-coating the active ingredients with a suitable protective material, or stratifying (layering) the active ingredients.

For instance, when the active medicinal agent, phenylephrine hydrochloride or phenylpropanolamine hydrochloride, each of which is a nasal decongestant, is used together with acetylsalicylic acid, the resulting combination is unstable and undergoes. a reaction which results in the formation of acetyl derivatives of the amine and degradation of the acetylsalicylic acid to salicylic acid. Thus, coated or layered compositions are used to prevent or reduce this degradation. Coating and/or layering techniques can be complex and expensive. Moreover, when it is desired to have an effervescent composition, these techniques are either not feasible or adversely affect effervescence of the solution.

Stable compositions of acetylsalicylic acid and the nasal decongestant, phenylephrine bitartrate, are known in the prior art. These compositions do not require layering or coating. Also known are stable compositions of the bitartrate and tartrate salts of phenylpropanolamine (nasal decongestants) together with acetylsalicylic acid (U.S. Patent No. 4,083,950), which likewise do not require layering or coating. The compositions disclosed in U.S. Patent 4,083,950 may also include the antihistamine, chlorpheniramine maleate, and an effervescent couple which particular compositions

MS-1381

are marketed as ALKA-SELTZER PLUS® (a registered trademark of Miles Laboratories, Inc., Elkhart, Indiana) cold medicine. Chlorpheniramine maleate also happens to be stable in the presence of acetylsalicylic acid.

Instability problems, however, can occur with many antihistamines in the presence of ASA. Recently it was proposed to employ the antihistamine, diphenhydramine hydrochloride, in combination with ASA since the most readily available salt of diphenhydramine is the hydrochloride salt. Diphenhydramine is an effective antihistamine, and thus it appeared to be useful in a cold medicine. However, the hydrochloride salt of diphenhydramine when used in conjunction with ASA results in considerable instability of the ASA due to a reaction which occurs between the ingredients, like the above-mentioned degradation that occurs when ASA is used in combination with phenylephrine hydrochloride or phenylpropanolamine hydrochloride. Thus, a suitable antihistamine salt of diphenhydramine was long sought.

## STATEMENT OF THE INVENTION

It was unexpectedly discovered that a substantially stable effervescent analgesic/antihistamine composition can be provided comprising a substantially uniform mixture of an effervescent couple and acetylsalicylic acid (ASA) in direct contact with the citrate salt of diphenhydramine (DPH citrate).

MS-1381

Therefore, the present invention provides for a substantially stable medicinal composition comprising a substantially uniform mixture of an effervescent couple, acetylsalicylic acid and diphenhydramine citrate in direct contact with each other, wherein these latter two components are present in the amount of about 50 to 99 weight percent acetylsalicylic acid and about 1 to 50 weight percent diphenhydramine citrate based upon the total weight of these latter two components thereby providing an effervescent system.

The use of such compositions in the production of medicaments for treating cold symptoms is also a subject of the invention. The composition is useful especially as nasal decongestant, against headache, body aches, muscular aches or pain. When referred to cold symptoms the symptoms described before are included in that meaning which, however, is not limited thereto.

## DETAILED DESCRIPTION OF THE INVENTION

The composition may include one or more other known active medicinal agents. More particularly, the present invention provides for such a stable composition containing an effervescent couple, i.e. a two-component system that liberates $CO_2$ when placed in water.

In producing the novel composition of the present invention, the various ingredients are appropriately blended together to form a substantially uniform mixture without the necessity of coating or otherwise preventing direct contact between the acetylsalicylic acid and the DPH citrate. Accordingly, the resulting composition is

MS 1381

essentially free of film-forming agents. The finished product form can be produced as powders, granules, capsules, or tablets. Although the amounts of acetylsalicylic acid and diphenhydramine citrate are not critical, the total amount of ingredients, as well as their relative amounts, is governed by the medicinal effects desired and by the standard pharmaceutical dosage. Thus, the amount of acetylsalicylic acid and DPH citrate present in the overall composition, which may contain other ingredients such as an effervescent couple, is about 50-99 weight percent acetylsalicylic acid and about 1-50 weight percent DPH citrate based on the total weight of the acetylsalicylic acid and the DPH citrate. Typically, a desirable pharmaceutical dose will contain approximately 80-1000 mg acetylsalicylic acid and approximately 19-77 mg DPH citrate.

A minor amount of excipients, lubricants, flavorings, and the like, as well as other active medicinal ingredients may be employed in the composition, as long as they do not substantially contribute to the instability of the acetylsalicylic acid. These active ingredients may be nasal decongestants, such as phenylpropanolamine bitartrate, phenylpropanolamine tartrate, or phenylepherine bitartrate, or other antihistamines such as chlorpheniramine maleate.

Also, an effervescent couple component is included in the present formulation to provide an effervescent system that liberates $CO_2$ when placed in water. Effervescent couples are well known and typically comprise an alkaline material capable of liberating carbon dioxide, such as sodium bicarbonate, sodium carbonate, potassium bicarbonate, potassium carbonate, and the like, together with an organic acid, such as citric acid, fumaric acid, adipic acid and the like.

The compositions included within the scope of the present invention have the advantage that the diphenhydramine citrate will not substantially react with acetylsalicylic acid when in direct contact with it. These compositions are substantially stable in that they have a storage stability essentially equal to similar compositions having the same ingredients but without any salt form of DPH. Thus, known techniques for preventing such contact, such as film-coating and/or tablet-layering, are obviated.

The invention is described in further detail in the examples below. These examples are intended to illustrate the preferred embodiments of the invention, and the invention is not intended to be limited thereby. In the Examples below, sodium bicarbonate and citric acid act as an effervescent couple.

## EXAMPLE 1

A first formulation comprising a mixture of acetylsalicylic acid, diphenhydramine hydrochloride, sodium bicarbonate and citric acid was fed to a tableting machine to form tablets each containing 325 mg of acetylsalicylic acid and 29 mg diphenhydramine hydrochloride. A second formulation comprising the above ingredients, except that diphenhydramine citrate was substituted for the diphenhydramine hydrochloride, was fed to a tableting machine to form tablets each comprising 325 mg of acetylsalicylic acid and 44 mg of diphenhydramine citrate. The second formulation and tablets contained about 88 weight percent of acetylsalicylic acid and about 12 weight percent diphenhydramine citrate based upon the total weight of the acetylsalicylic acid and diphenhydramine citrate. Tablets of each formulation according to Example I were then stored at 40°C. Samples were removed at intervals of 4 weeks and 13 weeks, and the sodium salicylate content per tablet of the samples was measured. Sodium salicylate is a decomposition product formed from the degradation of the acetylsalicylic acid in the presence of sodium ions from the sodium bicarbonate. The analysis first involves mixing the tablets in an aqueous HCl solution to convert the sodium salicylate into salicylic acid and NaCl. Next, the ingredients are extracted into chloroform. Ferric ammonium sulfate is then added. It reacts with the salicylic acid to produce a violet-colored compound. The color is typical of most reactions between phenolic type compounds and ferric ions. Since the color obeys Beer's law, a colorimetric measurement can be taken, the results of which correlate with the amount of salicylic acid present. The molecular weight of sodium salicylate is 160 and that of salicylic acid is 138. Thus, a conversion factor of 1.16 is used since 1.16 = 160/138. The results are reported in Table 1A below.

## TABLE 1A

### Sodium salicylate content in mg per tablet.

### Storage time at 40°C.

|   | Formulation | 4 Weeks | 13 Weeks |
|---|---|---|---|
| 1. | DPH HCl | 21.65 | 56.65 |
| 2. | DPH Citrate | 11.34 | 16.31 |

Another decomposition product is $CO_2$ from the effervescent couple. Tablets of each formulation according to Example I were packaged in twin-foil packets approximately 4.5 cm by 7 cm in size. The packets contain two tablets, side by side, and are airtight. Immediately after packaging, a packet has a thickness of approximately 0.5 cm. Stacks of packets, each having 10 packets piled one on top of another in a holder were stored at various temperatures in order to measure the extent of puffing caused as the amount of the $CO_2$ decomposition product increased. One set of stacks consisted of a 10-packet stack of the first formulation with DPH HCl and a 10-packet stack of the second formulation with DPH citrate stored at 40°C. Another set of stacks consisted of a 10-packet stack of the first formulation with DPH HCl and a 10-packet stack of the second formulation with DPH citrate stored at 50°C. The height of each 10-packet stack was measured initially and then at intervals of 4 weeks and 13 weeks (for those stored at 40°C) and at 1 week (for those stored at 50°C). The results are reported in Table 1B below.

## TABLE 1B

### Puffing Height in Centimeters

| | | | Storage Time at 50°C | | Storage Time at 40°C | |
|---|---|---|---|---|---|---|
| | Formulation | Initial | 1 Wk. | 4 Wks. | 13 Wks. | |
| 1. | DPH HCl | 4.9 | 17.5 | 10.7 | 17.5 | |
| 2. | DPH Citrate | 4.9 | 7.7 | 6.4 | 7.5 | |

It can be seen from the above data that tablets with diphenhydramine citrate in contact with acetylsalicylic acid have significantly improved storage stability as compared to tablets having the hydrochloride salt of diphenhydramine in contact with acetylsalicylic acid.

## EXAMPLE 2

A first formulation comprising about 9.7 weight percent acetylsalicylic acid, about 0.1 weight percent chlorpheniramine maleate (an antihistamine), about 0.8 weight percent phenylpropanolamine bitartrate (a decongestant), about 0.7 weight percent diphenhydramine hydrochloride, about 51.4 weight percent sodium bicarbonate, about 36.7 weight percent citric acid and about 0.5 weight percent flavorings was fed to a tableting machine to form tablets each comprising 325 mg of acetylsalicylic acid and 25 mg of diphenhydramine hydrochloride. A second formulation comprising the above ingredients, except that diphenhydramine citrate was substituted for the diphenhydramine hydrochloride, was fed to a tableting machine to form tablets each containing 325 mg of acetylsalicylic acid and 38.33 mg of diphenhydramine citrate. The second formulation and tablets contained about 89 weight percent acetylsalicylic acid and about 11 weight percent diphenhydramine citrate based upon the total weight of the acetylsalicylic acid and the diphenhydramine citrate. A third formulation comprising the above ingredients, except without any diphenhydramine salt, was fed to a tableting machine to form tablets each containing 325 mg acetylsalicylic acid. This third formulation was used as a control.

Tablets of each formulation were then stored at 40°C and at room temperature. Samples were removed at various time intervals, and the sodium salicylate content per tablet of each sample was measured in the same manner as in Example 1 above. The results are shown below in Table 2A below.

TABLE 2A

Sodium salicylate content in mg per tablet

| Formulation | Storage Time at 40°C | | Storage Time at Room Temperature | |
|---|---|---|---|---|
| | 4 Wks | 13 Wks | 26 Wks | 52 Wks |
| DPH HCl | 41.0 | 74.2 | 15.6 | 22.5 |
| DPH Citrate | 12.7 | 22.5 | 8.1 | 10.6 |
| No DPH salt | 12.1 | 19.8 | 7.9 | 10.2 |

Puffing data was obtained in the same manner as in Example 1 above. The results are reported in Table 2B below.

MS-1381

## TABLE 2B
### Puffing Height in Centimeters

| | | Storage Time at 50°C | Storage Time at 40°C | |
| --- | --- | --- | --- | --- |
| Formulation | Initial | 3 Days | 4 Wks. | 13 Wks. |
| DPH HCl | 5.2 | 11.8 | 14.0 | 17.5 |
| DPH Citrate | 4.6 | 7.1 | 6.0 | 6.0 |
| No DPH Salt | 5.0 | 7.4 | 6.0 | 6.5 |

The formulations contemplated by the present invention are substantially stable. By "substantially stable", it is intended to mean, as can be seen from the above data, that tablets with diphenhydramine citrate in direct contact with ASA have significantly improved storage stability as compared to tablets having the hydrochloride salt of diphenhydramine in direct contact with ASA. In fact, the storage stability of the tablets having acetylsalicylic acid in the presence of diphenhydramine citrate is essentially equal to the storage stability of the control tablets having acetylsalicylic acid in the absence of any diphenhydramine salt form.

The improved stability achieved by the diphenhydramine citrate provides a commercially acceptable effervescent system intended as in these examples for ingestion as a medicinal product suitable for relief of many of the symptoms connected with a cold or with hay fever.

MS-1381

What is Claimed is:

1.    A substantially stable medicinal composition comprising a substantially uniform mixture of an effervescent couple, acetylsalicylic acid and diphenhydramine citrate in direct contact with each other, characterized in that these latter two components are present in the amount of about 50 to 99 weight percent acetylsalicylic acid and about 1 to 50 weight percent diphenhydramine citrate based upon the total weight of these latter two components, thereby providing an effervescent system.

2.    The composition of Claim 1 further including another medicinal agent selected from the group consisting of phenylpropanolamine bitartrate, phenylpropanolamine tartrate, phenylepherine bitartrate, chlorpheniramine maleate, and mixtures thereof.

3.    The composition of Claim 1 or 2, wherein the effervescent couple consists essentially of an organic acid and an alkaline material.

4.    The composition of Claim 3, wherein the alkaline material is sodium bicarbonate, sodium carbonate, potassium bicarbonate, potassium carbonate, or a mixture thereof, and the organic acid is citric acid, fumaric acid, adipic acid, or a mixture thereof.

MS-1381 -EP

5. A stable pharmaceutical dosage form comprising a substantially uniform mixture of an effervescent couple, acetylsalicylic acid and diphenhydramine citrate in direct contact with each other, wherein these two latter components are present in the amount of about 80 to 1000 mg acetylsalicylic acid and about 19 to 77 mg diphenhydramine citrate, thereby providing an effervescent system.

6. A method for the preparation of a composition which method comprises admixing an effervescent couple, acetylsalicylic acid and diphenhydramine citrate in direct contact with each other, wherein the acetylsalicylic acid and the diphenhydramine citrate are present in the amount of 50 to 99 weight percent acetylsalicylic acid and from 1 to 50 weight percent diphenhydramine citrate based upon the total weight of these latter two components, thereby providing an effervescent system.

7. The method of Claim 6, further including compacting the admixture into a substantially stable, tablet dosage form.

8. A composition comprising an effervescent couple, about 50 to 99 weight percent acetylsalicylic acid and about 1 to 50 weight percent diphenhydramine citrate based on the total weight of these two latter components, wherein said composition is essentially free of film-forming agents and whereby the composition, when placed in $H_2O$, effervesces and liberates $CO_2$.

MS 1381

9.  Use of a substantially uniform mixture of an effervescent couple, acetylalicylic acid and diphenhydramine citrate in the production of medicinal compositions for treating cold symptoms.

MS 1381